(19) Europäisches Patentamt European Patent Office Office européen des brevets

(11) **EP 4 663 114 A2**

(12) **EUROPEAN PATENT APPLICATION**

(43) Date of publication:
**17.12.2025 Bulletin 2025/51**

(21) Application number: **25213314.5**

(22) Date of filing: **13.04.2022**

(51) International Patent Classification (IPC):
***A61B 5/024*** (2006.01)

(52) Cooperative Patent Classification (CPC):
**A61B 5/0075; A61B 5/02438; A61B 5/14546; A61B 5/14553; A61B 5/725;** A61B 5/6814; A61B 5/6826

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.04.2021 US 202163178120 P**

(62) Document number(s) of the earlier application(s) in accordance with Art. 76 EPC:
**22720226.4 / 4 304 455**

(71) Applicant: **Becton, Dickinson and Company**
**Franklin Lakes, NJ 07417-1880 (US)**

(72) Inventor: **BENNI, Paul, B.**
**Irvine, CA, 92614 (US)**

(74) Representative: **Venner, Julia Ann et al**
**Kilburn & Strode LLP**
**Lacon London**
**84 Theobalds Road**
**London WC1X 8NL (GB)**

Remarks:
This application was filed on 04.11.2025 as a divisional application to the application mentioned under INID code 62.

(54) **NIRS/TISSUE OXIMETRY BASED METHOD TO MEASURE ARTERIAL BLOOD OXYGEN SATURATION FROM PULSATILE HEMOGLOBIN WAVEFORMS**

(57)     A method and apparatus for non-invasively determining a tissue arterial oxygen saturation value of a tissue body is provided. The method includes: a) transmitting at least a first wavelength and a second wavelength of near-infrared light into a tissue body, the first wavelength different from the second wavelength; b) sensing the tissue body for the near-infrared light, and producing signals representative of the sensed near-infrared light; c) determining an AC component of a first tissue oxygen parameter using the signals; d) determining an AC component of a second tissue oxygen parameter using the signals; and e) determining a tissue arterial oxygen saturation value of a tissue body using the determined AC component of the first tissue oxygen parameter and the determined AC component of the second tissue oxygen parameter.

*FIG. 1*

## Description

This application claims priority to U.S. Patent Application No. 63/178,120 filed April 22, 2021, which is hereby incorporated by reference in its entirety.

BACKGROUND

1. Technical Field

The present disclosure relates to methods and apparatus for non-invasively determining biological tissue oxygen parameters in general, and to non-invasive methods and apparatus for non-invasively determining arterial tissue oxygen saturation levels in particular.

2. Background Information

Near-infrared spectroscopy (NIRS) is a spectrophotometric method of continually monitoring tissue oxygenation. The NIRS method is based on the principle that light in the near-infrared range (700 to 1,000 nm) can pass easily through skin, bone, and other tissues where it encounters hemoglobin located mainly within micro-circulation passages (e.g., capillaries, arterioles, and venules). Hemoglobin exposed to light in the near infra-red range has specific absorption spectra that varies depending on its oxidation state (i.e., oxyhemoglobin ($HbO_2$) and deoxyhemoglobin (Hb) each act as a distinct chromophore). By using light sources that transmit near-infrared light at specific different wavelengths, and measuring changes in transmitted or reflected light attenuation, concentration changes of the oxyhemoglobin ($HbO_2$) and deoxyhemoglobin (Hb) can be monitored.

A NIRS oximetry system typically includes a processor in communication with one or more sensors, each sensor having at least one light source and at least one light detector. Light emitted from the light source enters the subject's tissue. The emitted light (i.e., photons) travels different paths through the tissue from the light source to the detectors due to the high light scattering characteristics of tissue (wavelength dependent) and is attenuated as it passes through the tissue. More specifically, the attenuation of certain wavelengths of light is attributable to the presence of $HbO_2$ and Hb. By measuring the light attenuation between the emitted light and the light sensed at the respective light detector, concentration changes of $HbO_2$ and Hb can be determined.

Some NIRS sensors are known to have a "near" light detector and a "far" light detector. The near detector is positioned closer to the light source than the far detector. The path of light sensed by the near detector is understood to differ from the path of light sensed by the far detector. Very often the near detector is positioned to detect light that has primarily passed through tissue (e.g., scalp and skull tissue) overlying the organ of interest (e.g., the brain), and the far detector is positioned to detect light that has primarily passed through both the overlying tissue and organ tissue disposed beneath the overlying tissue. Although the light path between the light source and the near light detector differs from the light path between the light source and the far light detector, both paths may be characterized as being "banana shaped" (e.g., substantially elliptical), each having a different path length and depth of penetration. A general rule in NIRS sensor design is that the mean photon tissue interrogation depth of the banana shaped light path is one half (½) the distance between the sensor light source and respective detector.

Because NIRS technology mainly interrogates the microvasculature of tissue, which includes arterioles, venules, and capillaries, a NIRS tissue oxygen saturation ($StO_2$) measurement is made on a mixture of both venous and arterial blood. The mean ratio of this mixture for brain has been estimated to be about 70% venous to 30% arterial blood volume (e.g., Ito et al., Arterial fraction of cerebral blood volume in humans measured by positron emission tomography, Ann Nucl Med 2001; Apr. 15(2):111-6). Under prior art teachings, the same mean ratio of 70% venous to 30% arterial blood volume is typically applied to all regions of the body (e.g., Pang CC, Measurement of body venous tone, J Pharmacol Toxicol Methods 2000; 44(2):341-60). Therefore, to validate NIRS, oxygenation measurements of both venous and arterial blood need to be considered from the venous output and arterial input of a target organ.

An organ receives oxygenated blood from one or more arteries. After the tissue of the organ extracts oxygen from the arterial blood for metabolism in the capillary bed, blood returns to the heart by one or more veins. Depending on the organ's metabolism level, the hemoglobin oxygen saturation of venous blood is typically low and can vary depending on the particular organ. Smaller veins drain into larger veins and then into the superior vena cava (SVC) or inferior vena cava (IVC) into the heart's right atrium (RA) and right ventricle (RV). Blood drawn from these locations can be called "central" venous (CV) blood, which will have an oxygen saturation somewhere in between the highest and lowest individual venous oxygen saturations of the organs. Besides the usual organs, skin and skeletal muscle metabolize oxygen significantly as well, because of the skin's large surface area and skeletal muscle's large mass relative to the rest of the body. For the brain, arterial blood supply is primarily from the carotid arteries and the primary venous drainage is by the internal jugular vein / jugular bulb. The liver (or hepatic) venous oxygen saturation tends to be lower than the intestine/mesenteric portal vein oxygen saturation since the portal vein supplies low oxygenated blood to the liver. As stated above, a NIRS tissue oxygen saturation ($StO_2$) measurement performed as described above reflects a mixture of both venous and arterial blood, the ratio of venous and arterial blood is estimated under prior art devices, and the ratio can actually vary substantially from the estimated ratio.

What is needed is a non-invasive method and apparatus that can measure the tissue arterial blood saturation rather than rely on an estimation.

SUMMARY

According to the present disclosure, a method of non-invasively determining a tissue arterial oxygen saturation value of a tissue body is provided. The method includes: a) transmitting at least a first wavelength and a second wavelength of near-infrared light into a tissue body, the first wavelength different from the second wavelength; b) sensing the tissue body for the near-infrared light, and producing signals representative of the sensed near-infrared light; c) determining an AC component of a first tissue oxygen parameter using the signals; d) determining an AC component of a second tissue oxygen parameter using the signals; and e) determining a tissue arterial oxygen saturation value of a tissue body using the determined AC component of the first tissue oxygen parameter and the determined AC component of the second tissue oxygen parameter.

In any of the aspects or embodiments described above and herein, the signals produced representative of the sensed near-infrared light include first signals representative of the first wavelength of near-infrared light, the first signals having an AC component and a DC component, and second signals representative of the second wavelength of near-infrared light, the second signals having an AC component and a DC component. In addition the method may further include: a) processing the first signals to isolate an AC component of the first signals; b) determining an amplitude of the AC component of the first signals, wherein the step of determining the AC component of the first tissue oxygen parameter uses the determined amplitude of the AC component of the first signals; c) processing the second signals to isolate an AC component of the second signals; d) determining an amplitude of the AC component of the second signals, and wherein the step of determining the AC component of the second tissue oxygen parameter uses the determined amplitude of the AC component of the second signals.

In any of the aspects or embodiments described above and herein, the step of determining an amplitude of the AC component of the first signals may include determining a peak-to-peak amplitude of the AC component of the first signals, and the step of determining the AC component of the first tissue oxygen parameter of the tissue may use the determined peak-to-peak amplitude of the AC component of the first signals, and the step of determining an amplitude of the AC component of the second signals may include determining a peak-to-peak amplitude of the AC component of the second signals, and the step of determining the AC component of the second tissue oxygen parameter of the tissue may use the determined peak-to-peak amplitude of the AC component of the second signals.

In any of the aspects or embodiments described above and herein, the step of determining a peak-to-peak amplitude of the AC component of the first signals may include filtering the AC component of the first signals to determine a value representative of the peak-to-peak amplitude of the AC component of the first signals, and the step of determining a peak-to-peak amplitude of the AC component of the second signals may include filtering the AC component of the second signals to determine a value representative of the peak-to-peak amplitude of the AC component of the second signals.

In any of the aspects or embodiments described above and herein, the step of determining the AC component of the first tissue oxygen parameter may include determining a peak-to-peak amplitude of the AC component of the first tissue oxygen parameter, and the step of determining the AC component of the second tissue oxygen parameter may include determining a peak-to-peak amplitude of the AC component of the second tissue oxygen parameter.

In any of the aspects or embodiments described above and herein, the first tissue oxygen parameter may be oxyhemoglobin ($HbO_2$) and the second tissue oxygen parameter may be deoxyhemoglobin (Hb), and the step of determining the arterial oxygen saturation value may use a ratio of the peak-to-peak amplitude of the AC component of the first tissue oxygen parameter and a sum of the peak-to-peak amplitude of the AC component of the first tissue oxygen parameter and the peak-to-peak amplitude of the AC component of the second tissue oxygen parameter.

In any of the aspects or embodiments described above and herein, the step of processing the first signals to isolate the AC component of the first signals may include filtering the first signals to remove the DC component of the first signals, and the step of processing the second signals to isolate the AC component of the second signals may include filtering the second signals to remove the DC component of the second signals.

In any of the aspects or embodiments described above and herein, the step of determining the tissue arterial oxygen parameter value using the determined AC component of the first tissue oxygen parameter and the AC component of the second tissue oxygen parameter may include determining a peak-to peak value of the AC component of the first tissue oxygen parameter and a peak-to-peak value of the AC component of the second tissue oxygen parameter.

In any of the aspects or embodiments described above and herein, the first tissue oxygen parameter may be oxyhemoglobin ($HbO_2$) and the second tissue oxygen parameter may be deoxyhemoglobin (Hb), and the step of

EP 4 663 114 A2

determining the tissue arterial oxygen saturation may use a ratio of the first oxygen parameter and a sum of the first oxygen parameter and the second oxygen parameter.

In any of the aspects or embodiments described above and herein, the signals produced representative of the sensed near-infrared light may include first signals representative of the first wavelength of near-infrared light, and second signals representative of the second wavelength of near-infrared light, and the step of determining the AC component of the first tissue oxygen parameter may use the first signals, and the step of determining the AC component of the second tissue oxygen parameter may use the second signals.

In any of the aspects or embodiments described above and herein, the step of determining the AC component of the first tissue oxygen parameter may include determining an amplitude of the AC component of the first tissue oxygen parameter, and the step of determining the AC component of the second tissue oxygen parameter may include determining an amplitude of the AC component of the second tissue oxygen parameter, and the step of determining the tissue arterial oxygen saturation value may use the determined amplitude of the AC component of the first tissue oxygen parameter and the determined amplitude of the AC component of the second tissue oxygen parameter.

In any of the aspects or embodiments described above and herein, the step of determining the amplitude of the AC component of the first tissue oxygen parameter may include determining a peak-to-peak amplitude of the AC component of the first tissue oxygen parameter, and the step of determining the amplitude of the AC component of the second tissue oxygen parameter of the tissue may include determining a peak-to-peak amplitude of the AC component of the second tissue oxygen parameter of the tissue.

In any of the aspects or embodiments described above and herein, the first tissue oxygen parameter may be oxyhemoglobin ($HbO_2$) and the second tissue oxygen parameter may be deoxyhemoglobin (Hb), and the step of determining the arterial oxygen saturation value may use a ratio of the peak-to-peak amplitude of the AC component of the first tissue oxygen parameter and a sum of the peak-to-peak amplitude of the AC component of the first tissue oxygen parameter and the peak-to-peak amplitude of the AC component of the second tissue oxygen parameter.

In any of the aspects or embodiments described above and herein, the at least said first wavelength and said second wavelength may be transmitted using a light source from a sensor transducer having at least one near detector and at least one far detector, and the at least one near detector is located a first distance from the light source and the at least one far detector is located a second distance from the light source and the second distance is greater than the first distance, and the sensing step may utilize the at least one near detector and the at least one far detector.

In any of the aspects or embodiments described above and herein, the transmitting step and the sensing step may utilize a sensor transducer having a light source and a light detector, the sensor transducer configured to receive the tissue body in a manner such that the tissue body is disposed between the light source and the light detector and the transmitted near-infrared light is transmitted from the light source, through the tissue body in a direction toward the light detector.

According to another aspect of the present disclosure, an apparatus for non-invasively determining a tissue arterial oxygen saturation value of a tissue body is provided. The apparatus includes at least one sensor transducer and a controller. The at least one sensor transducer has a light source configured to produce at least a first wavelength and a second wavelength of near-infrared light, and at least one light detector is configured to sense the at least said first wavelength and said second wavelength of near-infrared light. The controller is in communication with the at least one sensor transducer. The controller includes at least one processor and a memory device configured to store instructions. The stored instructions when executed cause the controller to: a) control the light source to transmit at least a first wavelength and a second wavelength of near-infrared light into a tissue body, the first wavelength different from the second wavelength; b) control the at least one light detector to sense the tissue body for the near-infrared light, and produce signals representative of the sensed near-infrared light; c) determine an AC component of a first tissue oxygen parameter using the signals; d) determine an AC component of a second tissue oxygen parameter using the signals; and e) determine a tissue arterial oxygen saturation value of the tissue body using the determined AC component of the first tissue oxygen parameter and the determined AC component of the second tissue oxygen parameter.

In any of the aspects or embodiments described above and herein, the signals produced representative of the sensed near-infrared light may include first signals representative of the first wavelength of near-infrared light, the first signals having an AC component and a DC component, and second signals representative of the second wavelength of near-infrared light, the second signals having an AC component and a DC component, and the stored instructions when executed cause the controller to: a) process the first signals to isolate an AC component of the first signals; b) determine an amplitude of the AC component of the first signals, wherein the determination of the AC component of the first tissue oxygen parameter uses the determined amplitude of the AC component of the first signals; c) process the second signals to isolate an AC component of the second signals; and d) determine an amplitude of the AC component of the second signals, wherein the determination of the AC component of the second tissue oxygen parameter uses the determined amplitude of the AC component of the second signals.

In any of the aspects or embodiments described above and herein, the determination of the amplitude of the AC component of the first signals may include determining a peak-to-peak amplitude of the AC component of the first signals, and the determination of the AC component of the first tissue oxygen parameter of the tissue may use the determined peak-to-peak amplitude of the AC component of the first signals, and the determination of the amplitude of the AC component of the second signals may include determining a peak-to-peak amplitude of the AC component of the second signals, and the step of determination of the AC component of the second tissue oxygen parameter of the tissue may use the determined peak-to-peak amplitude of the AC component of the second signals.

In any of the aspects or embodiments described above and herein, the determination of the peak-to-peak amplitude of the AC component of the first signals may include filtering the AC component of the first signals to determine a value representative of the peak-to-peak amplitude of the AC component of the first signals, and the determination of the peak-to-peak amplitude of the AC component of the second signals may include filtering the AC component of the second signals to determine a value representative of the peak-to-peak amplitude of the AC component of the second signals.

In any of the aspects or embodiments described above and herein, the determination of the AC component of the first tissue oxygen parameter may include determining a peak-to-peak amplitude of the AC component of the first tissue oxygen parameter, and the determination of the AC component of the second tissue oxygen parameter of the tissue may include determining a peak-to-peak amplitude of the AC component of the second tissue oxygen parameter of the tissue.

In any of the aspects or embodiments described above and herein, the first tissue oxygen parameter may be oxyhemoglobin ($HbO_2$) and the second tissue oxygen parameter may be deoxyhemoglobin (Hb), and the determination of the arterial oxygen saturation value may use a ratio of the peak-to-peak amplitude of the AC component of the first tissue oxygen parameter and a sum of the peak-to-peak amplitude of the AC component of the first tissue oxygen parameter and the peak-to-peak amplitude of the AC component of the second tissue oxygen parameter.

In any of the aspects or embodiments described above and herein, the processing of the first signals to isolate the AC component of the first signals may include filtering the first signals to remove the DC component of the first signals, and the processing of the second signals to isolate the AC component of the second signals may include filtering the second signals to remove the DC component of the second signals.

In any of the aspects or embodiments described above and herein, the determination of the tissue arterial oxygen parameter value using the determined AC component of the first tissue oxygen parameter and the AC component of the second tissue oxygen parameter may include determining a peak-to peak value of the AC component of the first tissue oxygen parameter and a peak-to-peak value of the AC component of the second tissue oxygen parameter.

In any of the aspects or embodiments described above and herein, the first tissue oxygen parameter may be oxyhemoglobin ($HbO_2$) and the second tissue oxygen parameter may be deoxyhemoglobin (Hb), and the determination of the tissue arterial oxygen saturation may use a ratio of the first oxygen parameter and a sum of the first oxygen parameter and the second oxygen parameter.

In any of the aspects or embodiments described above and herein, the signals produced representative of the sensed near-infrared light may include first signals representative of the first wavelength of near-infrared light, and second signals representative of the second wavelength of near-infrared light, and the determination of the AC component of the first tissue oxygen parameter may use the first signals, and the determination of the AC component of the second tissue oxygen parameter may use the second signals.

In any of the aspects or embodiments described above and herein, the determination of the AC component of the first tissue oxygen parameter may include determining an amplitude of the AC component of the first tissue oxygen parameter, and the determination of the AC component of the second tissue oxygen parameter may include determining an amplitude of the AC component of the second tissue oxygen parameter, and the determination of the tissue arterial oxygen saturation value may use the determined amplitude of the AC component of the first tissue oxygen parameter and the determined amplitude of the AC component of the second tissue oxygen parameter.

In any of the aspects or embodiments described above and herein, the determination of the amplitude of the AC component of the first tissue oxygen parameter may include determining a peak-to-peak amplitude of the AC component of the first tissue oxygen parameter, and the determination of the amplitude of the AC component of the second tissue oxygen parameter of the tissue may include determining a peak-to-peak amplitude of the AC component of the second tissue oxygen parameter of the tissue.

In any of the aspects or embodiments described above and herein, the first tissue oxygen parameter may be oxyhemoglobin ($HbO_2$) and the second tissue oxygen parameter may be deoxyhemoglobin (Hb), and the determination of the arterial oxygen saturation value may use a ratio of the peak-to-peak amplitude of the AC component of the first tissue oxygen parameter and a sum of the peak-to-peak amplitude of the AC component of the first tissue oxygen parameter and the peak-to-peak amplitude of the AC component of the second tissue oxygen parameter.

In any of the aspects or embodiments described above and herein, the at least one detector of the at least one sensor transducer may include at least one near detector and at least one far detector, wherein the at least one near detector is located a first distance from the light source and the at least one far detector is located a second distance from the light source and the second distance is greater than the first distance.

In any of the aspects or embodiments described above and herein, the at least one sensor transducer may be configured to receive the tissue body in a manner such that the tissue body is disposed between the light source and the light detector and the transmitted near-infrared light is transmitted from the light source, through the tissue body in a direction toward the light detector.

According to another aspect of the present disclosure, a non-transitory computer-readable medium containing computer program instructions is provided. The computer program instructions are executable by the at least one computer processor to perform a method of non-invasively determining a tissue arterial oxygen saturation value of a tissue body, the method comprising: a) controlling a light source to transmit at least a first wavelength and a second wavelength of near-infrared light into a tissue body, the first wavelength different from the second wavelength; b) controlling at least one light detector to sense the tissue body for the near-infrared light, and producing signals representative of the sensed near-infrared light; c) determining an AC component of a first tissue oxygen parameter using the signals; d) determining an AC component of a second tissue oxygen parameter using the signals; and e) determining a tissue arterial oxygen saturation value of a tissue body using the determined AC component of the first tissue oxygen parameter and the determined AC component of the second tissue oxygen parameter.

The foregoing has outlined several aspects of the present invention in order that the detailed description of the invention that follows may be better understood. Additional features and advantages of the invention will be described hereinafter which form the subject of the claims of the invention.

BRIEF DESCRIPTION OF THE DRAWINGS

FIG. 1 is a diagrammatic of an embodiment of a present disclosure NIRS sensing system with sensor transducers applied to a subject's forehead.
FIG. 2 is a diagrammatic illustration of a sensor transducer in communication with a tissue surface disposed above a target organ (e.g., a brain).
FIG. 3A is a diagrammatic planar view of a sensor transducer having a light source, a near light detector, and a far light detector.
FIG. 3B is a diagrammatic side view of the sensor transducer shown in FIG. 3A.
FIG. 4 is a diagrammatic illustration of a sensor transducer in communication with a tissue surface (e.g., a digit).

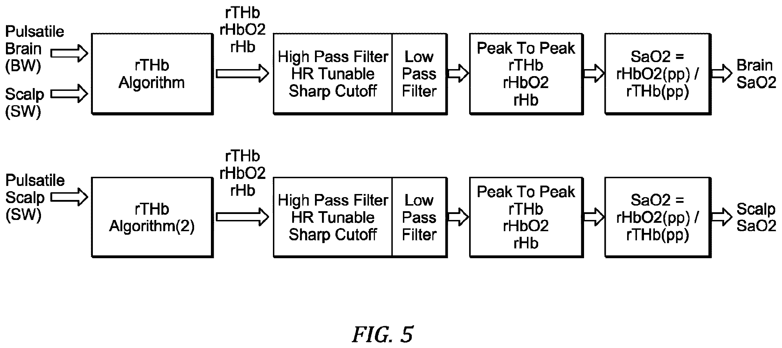

FIG. 5 is a diagrammatic illustration of signal processing used in some embodiments of the present disclosure.
FIG. 6 is a flowchart illustrating functions of an embodiment of the present disclosure.
FIG. 7 is a flowchart illustrating functions of an embodiment of the present disclosure.

DISCLOSURE OF THE INVENTION

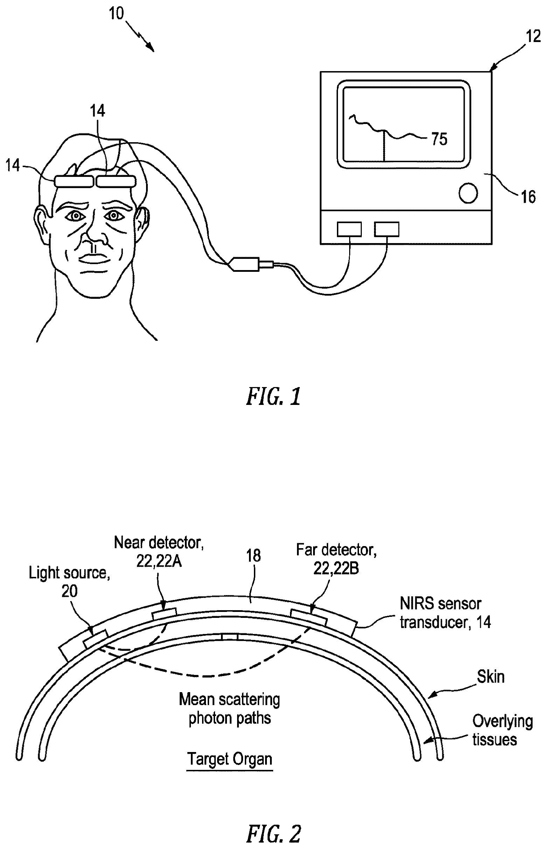

Referring to FIG. 1, the present method of and apparatus 10 for non-invasively determining an arterial tissue oxygen saturation includes a near infrared spectrophotometric (NIRS) tissue sensing device 12 (which may be referred to herein as a "NIRS oximeter 12") that includes one or more sensor transducers 14 and a controller 16. Each sensor transducer 14 is configured to selectively transmit light signals into the tissue (i.e., a tissue body) of a subject and to sense the transmitted light signals once they have passed through the tissue via transmittance or reflectance. The present disclosure is primarily described herein as including a NIRS cerebral oximeter to facilitate the description. The present disclosure is not, however, limited to including a NIRS cerebral oximeter. Oximeters configured to sense other tissue regions may be used alternatively; e.g., pulse oximeters configured to non-invasively sense digit or earlobe tissue. The NIRS oximeter 12 diagrammatically shown in FIG. 1 is a NIRS cerebral oximeter that includes a pair of sensor transducers 14 attached to a subject's forehead; e.g., to sense the right and left hemisphere of the subject's brain.

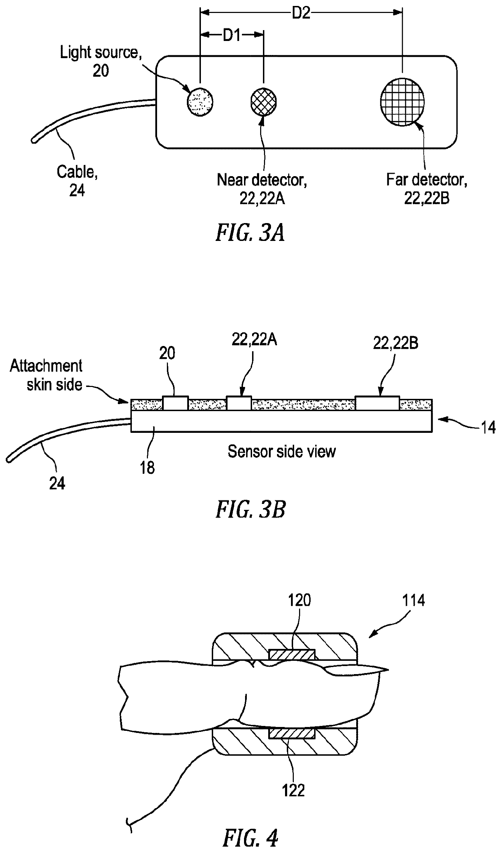

An example of an acceptable NIRS sensor transducer 14 is diagrammatically shown in FIG. 2. The sensor transducer 14, which may include a flexible body 18 that can be attached directly to a subject's tissue surface, includes one or more light sources 20 and one or more light detectors 22 mounted to or within the body 18. The light detectors may be described as a "near" detector 22A and a "far" detector 22B, where the terms "near" and "far" indicate the relative distances from the light source 20. FIGS. 3A and 3B provide a diagrammatic planar view and a side view of a sensor transducer 14 having a light source 20, a near light detector 22A, and a far light detector 22B. The near light detector 22A is separated from the light source 20 by a distance "D1" and the far light detector 22B is separated from the light source 20 by a distance "D2", where D2 is greater than D1 (D2 > D1). A disposable adhesive envelope or pad may be used to mount the sensor transducer 14 easily and securely to the subject's tissue surface. The light sources 20 may be light emitting diodes ("LEDs") that emit light at a narrow spectral bandwidth at predetermined wavelengths. The present disclosure is not, however, limited to using LEDs as a light source 20. A connector cable 24 may be used to connect the sensor transducer 14 components (e.g., lights source 20, light detectors 22, etc.) of each sensor transducer 14 directly or indirectly to the controller 16. The light detectors 22 are configured to sense light and produce signals representative of the sensed light. A non-limiting example of a light detector 22 type that can be used is a photodiode. Non-limiting examples of acceptable NIRS sensor transducers 14 are described in U.S. Patent Publication No. 2014/0171761 and U.S. Pat. No. 8,428,674, both of which are hereby incorporated by reference in their entirety. The above described NIRS sensor transducer 14 is a non-limiting example of a sensor transducer that may be used with the present disclosure. As stated herein, embodiments of the present disclosure may utilize NIRS oximeter 12 configurations other than a NIRS cerebral oximeter; e.g., a pulse oximeter. For those embodiments that use light transmitted through a digit or other tissue appendage, a NIRS sensor transducer 114 may have a finger mounting configuration (e.g., see FIG. 4) that includes a cuff configured to position a light source 120 on one side of the subject's digit and a light detector 122 on the opposite side of the subject's digit. Light emitted from the light source 120 passes through the digit via transmittance and is sensed by the light detector 122.

The controller 16 is in communication with at least some of the components within each NIRS sensor transducer 14 (e.g., the light source(s) 20, light detector(s) 22, etc.) to control and/or receive signals therefrom to perform the functions described herein. The controller 16 may include any type of computing device, computational circuit, processor(s), CPU, computer, or the like capable of executing a series of instructions that are stored in memory. The instructions may include an operating system, and/or executable software modules such as program files, system data, buffers, drivers, utilities, and the like. The executable instructions may apply to any functionality described herein to enable the NIRS oximeter 12 to accomplish the same algorithmically and/or coordination of device components. The controller 16 may include a single memory device or a plurality of memory devices. The present disclosure is not limited to any particular type of non-transitory memory device, and may include read-only memory, random access memory, volatile memory, nonvolatile memory, static memory, dynamic memory, flash memory, cache memory, and/or any device that stores digital information. The controller 16 may include, or may be in communication with, an input device that enables a user to enter data and/or instructions, and may include, or be in communication with, an output device configured, for example to display information (e.g., a visual display or a printer), or to transfer data, etc. Communications between the controller 16 and the NIRS oximeter 12 and its components may be via a hardwire connection or via a wireless connection. A person of skill in the art will recognize that portions of the controller 16 may assume various forms (e.g., digital signal processor, analog device, etc.) capable of performing the functions described herein.

It is known that near-infrared light passes through tissue readily and is absorbed by certain biological molecules disposed within the tissue. Near-infrared spectroscopy (NIRS) detects oxygenation changes in biological tissue (e.g., brain, muscle, or other organs) mainly at the microcirculation level (capillaries, arterioles, and venules) based on different absorption characteristics of certain chromophores (e.g., $HbO_2$, Hb) in the near-infrared spectrum (660 - 1000 nm). Average light penetration into the tissue is about 2-3 cm with sub-second time resolution. Relative changes of the concentrations of the chromophores (e.g., $HbO_2$, Hb) can be quantified by using the modified Beer-Lambert Law, which quantifies optical attenuation in a highly scattering medium like biological tissue. An example of a modified Beer-Lambert law is as follows:

$$A_\lambda = -\log(I/I_o)_\lambda = \alpha_\lambda * C * d * B + G \qquad \text{(Equation 1)}$$

where A is the optical attenuation in tissue at wavelength $\lambda$ (units: optical density or "OD"); $I_o$ is the incident light intensity (units: $W/cm^2$); $I$ is the detected light intensity; $\alpha_\lambda$ is the wavelength dependent absorption coefficient of the chromophore (units: $OD * cm^{-1} * \mu M^{-1}$); C is the concentration of chromophore (units: $\mu M$); $d$ is the light source 20 to detector distance (units: cm); $B$ is the light scattering differential pathlength factor (unit-less); and G is a factor relating to tissue geometry and scattering of light (units: OD).

Absolute measurement of chromophore concentration is very difficult because G is unknown. However, over a reasonable measuring period of several hours to days, G remains constant, allowing for the possibility of measuring relative changes of the respective chromophore from a zero reference baseline. Thus, if time $t_2$ is an arbitrary time after the start of the optical measurement at $t_1$ (baseline), differential attenuation $\Delta A$ can be calculated, canceling out the variables G and $I_o$, providing that they remain constant. The objective is to determine change in chromophore (e.g., Hb, $HbO_2$) concentration between time $t_1$ and $t_2$ (e.g., $\Delta C = C(t_2) - C(t_1)$) from $\Delta A$ based on the equation:

$$\Delta A = -\log(I_2/I_1)_\lambda = \alpha_\lambda * \Delta C * d * B \qquad \text{(Equation 2)}$$

NIRS algorithms designed to calculate the relative changes of more than one chromophore may use a multivariate form of Equation 2. Equation 3, shown below is a non-limiting example of a multivariate form of Equation 2:

$$\begin{bmatrix} -\log(I_2/I_1)_{\lambda 1}/(d*B_{\lambda 1}) \\ -\log(I_2/I_1)_{\lambda 2}/(d*B_{\lambda 2}) \\ \vdots \\ -\log(I_2/I_1)_{\lambda n}/(d*B_{\lambda n}) \end{bmatrix} = \begin{bmatrix} \alpha_{Hb\lambda 1} & \alpha_{HbO2\lambda 1} \\ \alpha_{Hb\lambda 2} & \alpha_{HbO2\lambda 2} \\ \vdots \\ \alpha_{Hb\lambda n} & \alpha_{HbO2\lambda n} \end{bmatrix} * \begin{bmatrix} \Delta Hb \\ \Delta HbO2 \\ \vdots \\ \vdots \end{bmatrix} \qquad \text{(Equation 3)}$$

where the left hand side of Equation 3 may be shown in abbreviated form as [ΔA/(d*B)], the first term on the right side of Equation 3 may be shown in abbreviated form as [α], and the second term on the right side of Equation 3 may be shown in abbreviated form as [Δc]; e.g., see Equations 4 and 5 below. To distinguish between and to compute relative changes in oxyhemoglobin ($\Delta HbO_2$) and deoxyhemoglobin ($\Delta Hb$), it is necessary to sense the tissue using a minimum of two different wavelengths. The units of $\Delta HbO_2$ and $\Delta HLb$ are in μmoles per liter of tissue (μM) which is determined from a dimensional analysis of equation 1. The differential pathlength factor B is wavelength dependent, leading to the exemplary expression:

$$B_{\lambda n} = B_{800} * k_{\lambda n}$$

The parameter $B_{800}$ is a user selected differential pathlength factor at 800 nm. The present disclosure is not limited to a differential pathlength factor at 800 nm, however. If the number of wavelengths used to sense the tissue is equal to the number of chromophores of interest, then Equation 3 can be solved in matrix form:

$$[\Delta c] = [\alpha]^{-1} * [\Delta A/(d * B)] \qquad \text{(Equation 4)}$$

where $[\alpha]^{-1}$ is the inverse matrix of [α], and [Δc] can be solved by Cramer's Rule. If the number of wavelengths used to sense the tissue is greater than the number of chromophores of interest, a least square multilinear regression method may be used to solve [Δc]:

$$[\Delta c] = (\alpha^T * \alpha)^{-1} * \alpha^T * [\Delta A/(d * B)] \qquad \text{(Equation 5)}$$

where $\alpha^T$ is the transpose of α, and $(\alpha^T * \alpha)^{-1}$ is the inverse matrix of the products of matrices $\alpha^T$ and α. In theory, the greater the number of wavelengths used, the greater the reduction of errors in the solution of Δc. The summation of $\Delta HbO_2$ and ΔHb give relative changes in total hemoglobin concentration ($\Delta TotalHb = \Delta HbO_2 + \Delta Hb$). The above-described methodology is a non-limiting example that can be used to determine tissue oxygenation parameters. Non-limiting alternative methodologies are disclosed in U.S. Patent Nos. 7,072,701; 8,396,526; 8,788,004; and 10,918,321, all of which are hereby incorporated by reference.

As stated above, tissue oxygen parameters (e.g., rTHb, $HbO_2$, Hb) concentrations (or changes in concentrations) determined using the methodology described above include both venous and arterial contributions. Prior art methods of determining the venous and arterial contributions independent of one another rely on an estimation of the venous to arterial ratio (e.g., 70% venous / 30% arterial), which estimate may not be accurate for all types of tissue. The present disclosure provides a methodology for determining the portion of tissue oxygen saturation attributable to arterial blood flow ($SaO_2$) using signals at the respective wavelengths associated with particular tissue oxygen parameters (e.g., rTHb, $HbO_2$, Hb).

For example in the manner described above, a NIRS oximeter 12 may be used to sense a subject's cerebral tissue to determine tissue oxygen parameter concentrations (or changes in concentrations); e.g., a device having one or more NIRS sensor transducers 14, each transducer 14 having one or more light sources 20 and one or more light detectors 22 mounted to or within the body 18 as shown in FIG. 1. The light detectors 22 may include a near light detector 22A and a far light detector 22B as described above. The light sources 20 emit predetermined wavelengths of light that interrogate the subject tissue, and the light detectors 22A, 22B sense the emitted light some distance(s) from the light source 20 after such light has passed through the subject's tissue. The light detectors 22A, 22B produce signals representative of the light originally emitted by the light source 20 (at a plurality of predetermined different wavelengths) and subsequently sensed by the respective light detector 22A, 22B. Those signals are subsequently processed (e.g., in the manner described above) to determine parameter values representative of the change in concentration (or the absolute concentration) of the respective tissue oxygen parameters (e.g., rTHb, $HbO_2$, Hb); e.g., see Equation 3 above.

The aforesaid determined parameter values are representative of the tissue oxygen parameters within the interrogated tissue as a function of time. As indicated above, the sensed tissue will include a microvasculature that includes arterioles, venules, and capillaries, and will therefore include arterial blood flow. Over time, the blood volume present within the tissue will vary as a result of the arterial blood flow. Hence, the signals produced by the light detectors 22 during tissue sensing and/or the parameter values determined from those signals may be described as having an "AC" component (i.e., a variable component) and a "DC" component (i.e., a substantially constant component). Embodiments of the present disclosure utilize the AC component to determine tissue oxygen saturation attributable to arterial blood. Pulse oximeters, in contrast, are known to use both AC and DC signals from different wavelengths.

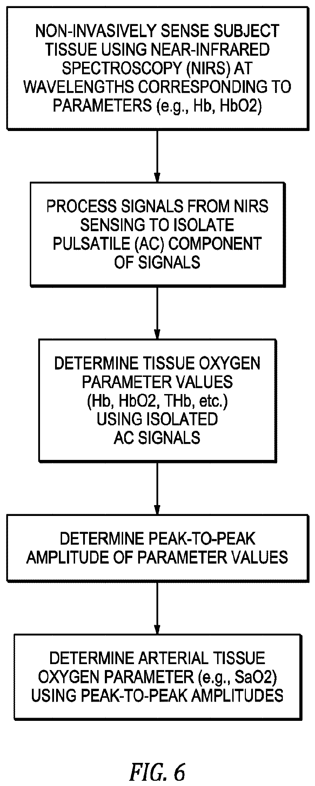

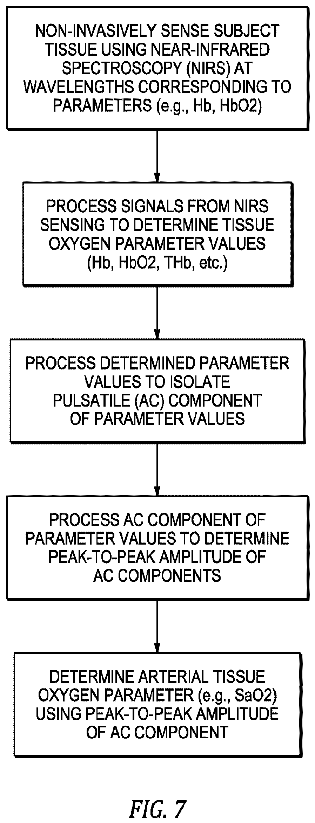

The AC component may be isolated by removing the DC component. Various different techniques can be used to isolate the AC component and the present disclosure is not limited to any particular technique. As an example, the signals produced by the light detectors 22 (which may be referred to as "raw signals") may be filtered using one or more high pass filters, low pass filters, band pass filters, or any combination thereof (see FIG. 6). For those embodiments that utilize a high pass filter, an acceptable example is a high pass filter having a sharp cutoff. In some embodiments, a tunable filter may be used; e.g., tunable to accommodate variations in heart rate. In those embodiments of the present disclosure that include a tunable filter, the NIRS oximeter 12 may include a device configured to determine the subject's heart rate or may be in communication with an independent device configured to determine the subject's heart rate. The aforesaid signal filtering may be performed within the controller 16 but is not required to be performed within the controller 16. The filtered signals representing the AC component may then be processed to determined tissue oxygen parameters (e.g., Hb, $HbO_2$, etc.) using an algorithmic approach such as shown in Equations 1-3. As an alternative (see FIG. 7), the aforesaid signals may be processed to produce parameter values and then those parameter values may be further processed to identify AC and DC components of the aforesaid parameter values and to isolate the AC component. Regardless of how it is isolated, the AC component may be characterized as being somewhat sinusoidal in shape with a peak-to-peak amplitude. The DC component may be discarded or may be utilized elsewhere.

As stated above, the present disclosure is configured to use the peak-to-peak amplitude of the AC component to determine the tissue arterial oxygen saturation (SaO2) level within the sensed tissue; e.g., via the stored instructions. More specifically, these embodiments of the present disclosure may utilize the peak-to-peak AC component attributable to $\Delta TotalHb$, $\Delta HbO_2$, and $\Delta Hb$ (i.e., either the raw signals from the light detectors 22 attributable to $\Delta TotalHb$, $\Delta HbO_2$, and $\Delta Hb$, or the parameter values for $\Delta TotalHb$, $\Delta HbO_2$, and Hb) using the following equation (where "P-P" refers to peak to peak):

$$SaO2(NIRS) = \frac{(P-P\ amplitude\ \Delta HbO2)}{(P-P\ amplitude\ \Delta Total\ Hb)} * K \qquad \text{(Equation 6)}$$

A non-limiting alternative to Equation 6 is provided below:

$$SaO2(NIRS) = \left(1 - \frac{P-P\ amplitude\ \Delta Hb}{P-P\ amplitude\ \Delta Total\ Hb}\right) * K \qquad \text{(Equation 7)}$$

In Equations 6 and 7 the "P-P amplitude $\Delta TotalHb$" refers to the sum of the peak to peak amplitudes of Hb and $HbO_2$.

Equations 6 and 7 are examples of mathematical equations that may be used to determine $SaO_2$ values using the peak-to-peak AC components, and the present disclosure is not limited to these exemplary equations. The term "K" as used in Equations 6 and 7 is a corrective factor that may not be required (e.g., its value can be set to 1). The term K reflects the possibility that some amount of error or shift may occur when using the Beer-Lambert law (e.g., modified as indicated above) to determine the concentration of the respective chromophores based on their absorption of particular wavelengths of light.

The term K may be determined by a variety of different techniques, and therefore the present disclosure is not limited to any particular technique. An example of an acceptable technique involves producing a clinically sufficient amount of empirical $SaO_2$ data produced using a NIRS oximeter (i.e., "$SaO_2$ (NIRS)") and using an alternative sensing technology device such as a CO-oximeter (i.e., "$SaO_2$ (CO-ox REF)") on the same or substantially similar test tissue. The $SaO_2$ (CO-ox REF) data may be determined using the following equation:

$$SaO2(co - ox\ REF) = \frac{(P-P\ amplitude\ \Delta HbO2)}{(P-P\ amplitude\ \Delta Total\ Hb)} * K \qquad \text{(Equation 8)}$$

Equation 8 represents an example of an equation or technique that can be used to determine $SaO_2$ (CO-ox REF) and the present disclosure is not limited thereto. The respective $SaO_2$ data determined using the two techniques can subsequently be comparatively analyzed to ascertain the level of agreement between the data, and to determine a corrective factor (e.g., "K"), if necessary, to establish agreement there between; e.g., a "best fit". In some embodiments, a clinically sufficient amount of $SaO_2$ (NIRS) data and $SaO_2$ (CO-ox REF) data may be collected and organized using an X-Y scatterplot (e.g., with $SaO_2$ (CO-ox REF) on the X-axis and $SaO_2$ (NIRS) data on the Y-axis). The analysis of the data may include a regression technique to determine the relationship between the $SaO_2$ (CO-ox REF) data and the $SaO_2$ (NIRS) data and the term K representative of that relationship. A scatterplot analysis is a non-limiting example of a technique that may be used, and the present disclosure is not limited thereto. In some embodiments, the determination of a K value may take into consideration additional factors, such as the raw signals attributable to $\Delta TotalHb$, $\Delta HbO_2$, and $\Delta Hb$, light source 20 to light detector 22 distances, and the like; i.e.:

$$K = function(\Delta TotalHb, HbO_2, and\ or\ raw\ signals\ @diff\ \lambda s, LS$$
$$- LD\ separation, etc.)$$

From the above, it can be seen that the term K may be a constant. The term K may alternatively be an empirically derived algorithmic calibrating relationship; e.g., a linear equation, a polynomial equation, a non-linear equation, etc.

The isolated AC component may be quantitatively analyzed to determine the peak-to-peak amplitude using a variety of different techniques. For example, the AC component may be processed using a root mean square (RMS) filter to provide a value that is proportional to the peak-to-peak amplitude of the respective AC component; e.g., the AC components respectively associated with $\Delta TotalHLb$, $\Delta HbO_2$, $\Delta Hb$, etc. When using an RMS filter, the peak-to-peak values of these respective AC components (as used in the determination of $SaO_2$) may be determined using the following equation:

$$P - P \ amplitude \ value = RMS * J \qquad \text{(Equation 9)}$$

where the term "J" is a constant or empirically determined corrective factor. As stated above, an RMS filter is an example of a means to determine a signal peak-to-peak amplitude value, and the present disclosure is not limited thereto.

As indicated above, the present disclosure may utilize different types of NIRS oximeters 12; e.g., cerebral oximeters, pulse oximeters, and any other NIRS sensing device operable to emit into a body of tissue and collect the emitted light reflected from the tissue, or the emitted light transmitted through the tissue, or any combination thereof. A non-limiting specific example of a NIRS cerebral oximeter is described above that utilizes sensor transducer 14 having a light source 20, one or more near light detectors 22A, and one or more far light detectors 22B (see FIGS. 2-3B). A benefit of the present disclosure using such a NIRS oximeter 12 stems from the different tissue bodies sensed as a result of collecting signal data using the spaced apart near and far light detectors 22A, 22B. As described above, the path of emitted light sensed by the near light detector 22A is understood to differ from the path of emitted light sensed by the far light detector 22B; i.e., the depth of tissue light interrogation sensed by the near light detector 22A is less than the depth of tissue light interrogation sensed by the far light detector 22B. Embodiments of the present disclosure that utilize a NIRS oximeter 12 with one or more NIRS sensor transducers 14 having near and far light detectors 22A, 22B can determine arterial tissue oxygen parameters for two different tissue bodies; e.g., arterial tissue oxygen parameters for a scalp and skull tissue region as well as arterial tissue oxygen parameters for a brain tissue region. The present disclosure is not limited to a NIRS cerebral oximeter, however. As indicated above, the present disclosure can utilize a finger mounted NIRS sensor transducer 114 (see FIG. 4) that includes a light source 120 disposed on one side of the subject's digit and a light detector 122 disposed on the opposite side of the subject's digit. Light emitted from the light source 120 passes through the digit via transmittance and is sensed by the light detector 122.

A benefit provided by some embodiments of the present disclosure is that they provide greater utility that currently available devices. Embodiments of the present disclosure that include a NIRS cerebral oximeter or the like that is configured to determine a tissue oxygen saturation value ($StO_2$) that includes contributions from both venous and arterial blood. The present disclosure can provide both $StO_2$ data and $SaO_2$ data; i.e., provide both $StO_2$ data and $SaO_2$ using a single device without the need to use a venous/arterial blood ratio estimation.

The present disclosure system 10 and method utilizing the AC component of light detector 22 signals and/or the AC component of determined tissue oxygen parameters (e.g., $HbO_2$, $Hb$) to determine $SaO_2$ data, are also understood to provide $SaO_2$ data with enhanced accuracy. Prior art systems for determining $SaO_2$ data (e.g., pulse oximeters) typically use light at a first wavelength (e.g., in the range of 660nm to 690nm) on one side of the hemoglobin isobestic point (i.e., the point where the light absorption of $HbO_2$ and $Hb$ are about the same ~ 805nm) and a second wavelength (e.g., in the range of 880nm to 940nm) on the opposite side of the isobestic point. As indicated above, the attenuation of light traveling through the tissue and the interrogation depth of that light is wavelength dependent at least in part due to pathlength factor "B". Hence, a tissue body interrogated by light at a first wavelength may not be the exact same tissue body interrogated by a light at a second, different wavelength, even if both wavelengths of light are emitted from and collected at the same light source 20 / light detector 22 combination. Embodiments of the present disclosure utilizing the AC component of signals and/or determined tissue oxygen parameter values can utilize wavelengths of light outside the aforesaid typical wavelength ranges (660-690 nm and 880-940 nm) on opposite sides of the isobestic point (~805nm) to determine $SaO_2$ data. More specifically, these embodiments may use wavelengths of light in the range of about 690nm - 810nm on one side of the isobestic point and may use wavelengths of light in the range of about 880nm - 810nm on the opposite side of the isobestic point. In a specific example, using the present disclosure, $SaO_2$ data can be produced using a first wavelength of light at about 770 nm and at about 870nm. As a result, the present disclosure permits arterial tissue oxygen parameters (e.g., $SaO_2$, $HbO_2$, $Hb$) to be determined using wavelengths of light that are closer to one another than is the case using prior art methods. Hence, any differences in light attenuation and interrogation depth between a common light source 20 and light detector 22 attributable to wavelength dependent factors (e.g., pathlength factor "B") are decreased due to the closer proximity of the interrogating wavelengths; i.e., there is a greater likelihood that the tissue body interrogated by one wavelength more closely coincides with the tissue body interrogated by the other wavelength.

As indicated above, the functionality described herein may be implemented, for example, in hardware, software tangibly embodied in a computer-readable medium, firmware, or any combination thereof. In some embodiments, at least a portion of the functionality described herein may be implemented in one or more computer programs. Each such computer program may be implemented in a computer program product tangibly embodied in non-transitory signals in a machine-readable storage device for execution by a computer processor. Method steps of the present disclosure may be performed by a computer processor executing a program tangibly embodied on a computer-readable medium to perform functions of the present disclosure by operating on input and generating output. Each computer program within the scope of the present claims below may be implemented in any programming language, such as assembly language, machine language, a high-level procedural programming language, of an object-oriented programming language. The programming language may, for example, be a compiled or interpreted programming language.

While the principles of the disclosure have been described above in connection with specific apparatuses and methods, it is to be clearly understood that this description is made only by way of example and not as limitation on the scope of the disclosure. Specific details are given in the above description to provide a thorough understanding of the embodiments. However, it is understood that the embodiments may be practiced without these specific details.

It is noted that the embodiments may be described as a process which is depicted as a flowchart, a flow diagram, a block diagram, etc. Although any one of these structures may describe the operations as a sequential process, many of the operations can be performed in parallel or concurrently. In addition, the order of the operations may be rearranged. A process may correspond to a method, a function, a procedure, a subroutine, a subprogram, etc.

The singular forms "a," "an," and "the" refer to one or more than one, unless the context clearly dictates otherwise. For example, the term "comprising a specimen" includes single or plural specimens and is considered equivalent to the phrase "comprising at least one specimen." The term "or" refers to a single element of stated alternative elements or a combination of two or more elements unless the context clearly indicates otherwise. As used herein, "comprises" means "includes." Thus, "comprising A or B," means "including A or B, or A and B," without excluding additional elements.

It is noted that various connections are set forth between elements in the present description and drawings (the contents of which are included in this disclosure by way of reference). It is noted that these connections are general and, unless specified otherwise, may be direct or indirect and that this specification is not intended to be limiting in this respect. Any reference to attached, fixed, connected or the like may include permanent, removable, temporary, partial, full and/or any other possible attachment option.

No element, component, or method step in the present disclosure is intended to be dedicated to the public regardless of whether the element, component, or method step is explicitly recited in the claims. No claim element herein is to be construed under the provisions of 35 U.S.C. 112(f) unless the element is expressly recited using the phrase "means for." As used herein, the terms "comprises", "comprising", or any other variation thereof, are intended to cover a non-exclusive inclusion, such that a process, method, article, or apparatus that comprises a list of elements does not include only those elements but may include other elements not expressly listed or inherent to such process, method, article, or apparatus.

While various inventive aspects, concepts and features of the disclosures may be described and illustrated herein as embodied in combination in the exemplary embodiments, these various aspects, concepts, and features may be used in many alternative embodiments, either individually or in various combinations and sub-combinations thereof. Unless expressly excluded herein all such combinations and sub-combinations are intended to be within the scope of the present application. Still further, while various alternative embodiments as to the various aspects, concepts, and features of the disclosures--such as alternative materials, structures, configurations, methods, devices, and components, and so on--may be described herein, such descriptions are not intended to be a complete or exhaustive list of available alternative embodiments, whether presently known or later developed. Those skilled in the art may readily adopt one or more of the inventive aspects, concepts, or features into additional embodiments and uses within the scope of the present application even if such embodiments are not expressly disclosed herein. For example, in the exemplary embodiments described above within the Detailed Description portion of the present specification, elements may be described as individual units and shown as independent of one another to facilitate the description. In alternative embodiments, such elements may be configured as combined elements.

Additionally, even though some features, concepts, or aspects of the disclosures may be described herein as being a preferred arrangement or method, such description is not intended to suggest that such feature is required or necessary unless expressly so stated. Still further, exemplary, or representative values and ranges may be included to assist in understanding the present application, however, such values and ranges are not to be construed in a limiting sense and are intended to be critical values or ranges only if so expressly stated.

Examples are set out in the following list of numbered clauses:

1. A method of non-invasively determining a tissue arterial oxygen saturation value of a tissue body, comprising:

transmitting at least a first wavelength and a second wavelength of near-infrared light into a tissue body, the first wavelength different from the second wavelength;

sensing the tissue body for the near-infrared light, and producing signals representative of the sensed near-infrared light;
determining an AC component of a first tissue oxygen parameter using the signals;
determining an AC component of a second tissue oxygen parameter using the signals; and
determining a tissue arterial oxygen saturation value of a tissue body using the determined AC component of the first tissue oxygen parameter and the determined AC component of the second tissue oxygen parameter.

2. The method of clause 1, wherein the signals produced representative of the sensed near-infrared light include first signals representative of the first wavelength of near-infrared light, the first signals having an AC component and a DC component, and second signals representative of the second wavelength of near-infrared light, the second signals having an AC component and a DC component; and
the method further comprising:

processing the first signals to isolate an AC component of the first signals;
determining an amplitude of the AC component of the first signals;
wherein the step of determining the AC component of the first tissue oxygen parameter uses the determined amplitude of the AC component of the first signals;
processing the second signals to isolate an AC component of the second signals;
determining an amplitude of the AC component of the second signals; and
wherein the step of determining the AC component of the second tissue oxygen parameter uses the determined amplitude of the AC component of the second signals.

3. The method of clause 2, wherein the step of determining an amplitude of the AC component of the first signals includes determining a peak-to-peak amplitude of the AC component of the first signals, and the step of determining the AC component of the first tissue oxygen parameter of the tissue uses the determined peak-to-peak amplitude of the AC component of the first signals; and
wherein the step of determining an amplitude of the AC component of the second signals includes determining a peak-to-peak amplitude of the AC component of the second signals, and the step of determining the AC component of the second tissue oxygen parameter of the tissue uses the determined peak-to-peak amplitude of the AC component of the second signals.

4. The method of clause 3, wherein the step of determining a peak-to-peak amplitude of the AC component of the first signals includes filtering the AC component of the first signals to determine a value representative of the peak-to-peak amplitude of the AC component of the first signals; and
wherein the step of determining a peak-to-peak amplitude of the AC component of the second signals includes filtering the AC component of the second signals to determine a value representative of the peak-to-peak amplitude of the AC component of the second signals.

5. The method of clause 3, wherein the step of determining the AC component of the first tissue oxygen parameter includes determining a peak-to-peak amplitude of the AC component of the first tissue oxygen parameter; and
wherein the step of determining the AC component of the second tissue oxygen parameter includes determining a peak-to-peak amplitude of the AC component of the second tissue oxygen parameter.

6. The method of clause 5, wherein the first tissue oxygen parameter is oxyhemoglobin ($HbO_2$) and the second tissue oxygen parameter is deoxyhemoglobin (Hb); and
wherein the step of determining the arterial oxygen saturation value uses a ratio of the peak-to-peak amplitude of the AC component of the first tissue oxygen parameter and a sum of the peak-to-peak amplitude of the AC component of the first tissue oxygen parameter and the peak-to-peak amplitude of the AC component of the second tissue oxygen parameter.

7. The method of clause 2, wherein the step of processing the first signals to isolate the AC component of the first signals includes filtering the first signals to remove the DC component of the first signals; and
wherein the step of processing the second signals to isolate the AC component of the second signals includes filtering the second signals to remove the DC component of the second signals.

8. The method of clause 1, wherein the step of determining the tissue arterial oxygen parameter value using the determined AC component of the first tissue oxygen parameter and the AC component of the second tissue oxygen parameter includes determining a peak-to peak value of the AC component of the first tissue oxygen parameter and a peak-to-peak value of the AC component of the second tissue oxygen parameter.

9. The method of clause 1, wherein the first tissue oxygen parameter is oxyhemoglobin ($HbO_2$) and the second tissue oxygen parameter is deoxyhemoglobin (Hb); and
wherein the step of determining the tissue arterial oxygen saturation uses a ratio of the first oxygen parameter and a sum of the first oxygen parameter and the second oxygen parameter.

10. The method of clause 1, wherein the signals produced representative of the sensed near-infrared light include first signals representative of the first wavelength of near-infrared light, and second signals representative of the second wavelength of near-infrared light; and
wherein the step of determining the AC component of the first tissue oxygen parameter uses the first signals, and the step of determining the AC component of the second tissue oxygen parameter uses the second signals.

11. The method of clause 10, wherein the step of determining the AC component of the first tissue oxygen parameter includes determining an amplitude of the AC component of the first tissue oxygen parameter; and

wherein the step of determining the AC component of the second tissue oxygen parameter includes determining an amplitude of the AC component of the second tissue oxygen parameter; and
wherein the step of determining the tissue arterial oxygen saturation value uses the determined amplitude of the AC component of the first tissue oxygen parameter and the determined amplitude of the AC component of the second tissue oxygen parameter.

12. The method of clause 11, wherein the step of determining the amplitude of the AC component of the first tissue oxygen parameter includes determining a peak-to-peak amplitude of the AC component of the first tissue oxygen parameter; and
wherein the step of determining the amplitude of the AC component of the second tissue oxygen parameter of the tissue includes determining a peak-to-peak amplitude of the AC component of the second tissue oxygen parameter of the tissue.

13. The method of clause 12, wherein the first tissue oxygen parameter is oxyhemoglobin ($HbO_2$) and the second tissue oxygen parameter is deoxyhemoglobin (Hb); and
wherein the step of determining the arterial oxygen saturation value uses a ratio of the peak-to-peak amplitude of the AC component of the first tissue oxygen parameter and a sum of the peak-to-peak amplitude of the AC component of the first tissue oxygen parameter and the peak-to-peak amplitude of the AC component of the second tissue oxygen parameter.

14. The method of clause 1, wherein the at least said first wavelength and said second wavelength are transmitted using a light source from a sensor transducer having at least one near detector and at least one far detector, wherein the at least one near detector is located a first distance from the light source and the at least one far detector is located a second distance from the light source and the second distance is greater than the first distance; and
wherein the sensing step utilizes the at least one near detector and the at least one far detector.

15. The method of clause 1, wherein the transmitting step and the sensing step utilize a sensor transducer having a light source and a light detector, the sensor transducer configured to receive the tissue body in a manner such that the tissue body is disposed between the light source and the light detector and the transmitted near-infrared light is transmitted from the light source, through the tissue body in a direction toward the light detector.

16. An apparatus for non-invasively determining a tissue arterial oxygen saturation value of a tissue body, comprising:

at least one sensor transducer having a light source configured to produce at least a first wavelength and a second wavelength of near-infrared light, and at least one light detector configured to sense the at least said first wavelength and said second wavelength of near-infrared light; and
a controller in communication with the at least one sensor transducer, the controller including at least one processor and a memory device configured to store instructions, the stored instructions when executed cause the controller to:

control the light source to transmit at least a first wavelength and a second wavelength of near-infrared light into a tissue body, the first wavelength different from the second wavelength;

control the at least one light detector to sense the tissue body for the near-infrared light, and produce signals representative of the sensed near-infrared light;
determine an AC component of a first tissue oxygen parameter using the signals;
determine an AC component of a second tissue oxygen parameter using the signals; and
determine a tissue arterial oxygen saturation value of the tissue body using the determined AC component of the first tissue oxygen parameter and the determined AC component of the second tissue oxygen parameter.

17. The apparatus of clause 16, wherein the signals produced representative of the sensed near-infrared light include first signals representative of the first wavelength of near-infrared light, the first signals having an AC component and a DC component, and second signals representative of the second wavelength of near-infrared light, the second signals having an AC component and a DC component; and
the stored instructions when executed cause the controller to:

process the first signals to isolate an AC component of the first signals;
determine an amplitude of the AC component of the first signals, wherein the determination of the AC component of the first tissue oxygen parameter uses the determined amplitude of the AC component of the first signals;
process the second signals to isolate an AC component of the second signals; and
determine an amplitude of the AC component of the second signals, wherein the determination of the AC component of the second tissue oxygen parameter uses the determined amplitude of the AC component of the second signals.

18. The apparatus of clause 17, wherein the determination of the amplitude of the AC component of the first signals includes determining a peak-to-peak amplitude of the AC component of the first signals, and the determination of the AC component of the first tissue oxygen parameter of the tissue uses the determined peak-to-peak amplitude of the AC component of the first signals; and
wherein the determination of the amplitude of the AC component of the second signals includes determining a peak-to-peak amplitude of the AC component of the second signals, and the step of determination of the AC component of the second tissue oxygen parameter of the tissue uses the determined peak-to-peak amplitude of the AC component of the second signals.

19. The apparatus of clause 18, wherein the determination of the peak-to-peak amplitude of the AC component of the first signals includes filtering the AC component of the first signals to determine a value representative of the peak-to-peak amplitude of the AC component of the first signals; and
wherein the determination of the peak-to-peak amplitude of the AC component of the second signals includes filtering the AC component of the second signals to determine a value representative of the peak-to-peak amplitude of the AC component of the second signals.

20. The apparatus of clause 18, wherein the determination of the AC component of the first tissue oxygen parameter includes determining a peak-to-peak amplitude of the AC component of the first tissue oxygen parameter; and
wherein the determination of the AC component of the second tissue oxygen parameter of the tissue includes determining a peak-to-peak amplitude of the AC component of the second tissue oxygen parameter of the tissue.

21. The apparatus of clause 20, wherein the first tissue oxygen parameter is oxyhemoglobin ($HbO_2$) and the second tissue oxygen parameter is deoxyhemoglobin (Hb); and
wherein the determination of the arterial oxygen saturation value uses a ratio of the peak-to-peak amplitude of the AC component of the first tissue oxygen parameter and a sum of the peak-to-peak amplitude of the AC component of the first tissue oxygen parameter and the peak-to-peak amplitude of the AC component of the second tissue oxygen parameter.

22. The apparatus of clause 17, wherein the processing of the first signals to isolate the AC component of the first signals includes filtering the first signals to remove the DC component of the first signals; and
wherein the processing of the second signals to isolate the AC component of the second signals includes filtering the second signals to remove the DC component of the second signals.

23. The apparatus of clause 16, wherein the determination of the tissue arterial oxygen parameter value using the determined AC component of the first tissue oxygen parameter and the AC component of the second tissue oxygen parameter includes determining a peak-to peak value of the AC component of the first tissue oxygen parameter and a peak-to-peak value of the AC component of the second tissue oxygen parameter.

24. The apparatus of clause 16, wherein the first tissue oxygen parameter is oxyhemoglobin ($HbO_2$) and the second tissue oxygen parameter is deoxyhemoglobin (Hb); and
wherein the determination of the tissue arterial oxygen saturation uses a ratio of the first oxygen parameter and a sum of the first oxygen parameter and the second oxygen parameter.

25. The apparatus of clause 16, wherein the signals produced representative of the sensed near-infrared light include first signals representative of the first wavelength of near-infrared light, and second signals representative of the second wavelength of near-infrared light; and
wherein the determination of the AC component of the first tissue oxygen parameter uses the first signals, and the determination of the AC component of the second tissue oxygen parameter uses the second signals.

26. The apparatus of clause 25, wherein the determination of the AC component of the first tissue oxygen parameter includes determining an amplitude of the AC component of the first tissue oxygen parameter; and

wherein the determination of the AC component of the second tissue oxygen parameter includes determining an amplitude of the AC component of the second tissue oxygen parameter; and
wherein the determination of the tissue arterial oxygen saturation value uses the determined amplitude of the AC component of the first tissue oxygen parameter and the determined amplitude of the AC component of the second tissue oxygen parameter.

27. The apparatus of clause 26, wherein the determination of the amplitude of the AC component of the first tissue oxygen parameter includes determining a peak-to-peak amplitude of the AC component of the first tissue oxygen parameter; and
wherein the determination of the amplitude of the AC component of the second tissue oxygen parameter of the tissue includes determining a peak-to-peak amplitude of the AC component of the second tissue oxygen parameter of the tissue.

28. The apparatus of clause 27, wherein the first tissue oxygen parameter is oxyhemoglobin ($HbO_2$) and the second tissue oxygen parameter is deoxyhemoglobin (Hb); and
wherein the determination of the arterial oxygen saturation value uses a ratio of the peak-to-peak amplitude of the AC component of the first tissue oxygen parameter and a sum of the peak-to-peak amplitude of the AC component of the first tissue oxygen parameter and the peak-to-peak amplitude of the AC component of the second tissue oxygen parameter.

29. The apparatus of clause 16, wherein the at least one detector of the at least one sensor transducer includes at least one near detector and at least one far detector, wherein the at least one near detector is located a first distance from the light source and the at least one far detector is located a second distance from the light source and the second distance is greater than the first distance.

30. The apparatus of clause 16, wherein the at least one sensor transducer is configured to receive the tissue body in a manner such that the tissue body is disposed between the light source and the light detector and the transmitted near-infrared light is transmitted from the light source, through the tissue body in a direction toward the light detector.

31. A non-transitory computer-readable medium containing computer program instructions, wherein the computer program instructions are executable by the at least one computer processor to perform a method of non-invasively determining a tissue arterial oxygen saturation value of a tissue body, the method comprising:

controlling a light source to transmit at least a first wavelength and a second wavelength of near-infrared light into a tissue body, the first wavelength different from the second wavelength;
controlling at least one light detector to sense the tissue body for the near-infrared light, and producing signals representative of the sensed near-infrared light;
determining an AC component of a first tissue oxygen parameter using the signals;
determining an AC component of a second tissue oxygen parameter using the signals; and
determining a tissue arterial oxygen saturation value of a tissue body using the determined AC component of the first tissue oxygen parameter and the determined AC component of the second tissue oxygen parameter.

**Claims**

1. A method of non-invasively determining a tissue arterial oxygen saturation value of a tissue body, comprising:

   transmitting at least a first wavelength and a second wavelength of near-infrared light into a tissue body, the first wavelength different from the second wavelength;
   sensing the tissue body for the near-infrared light, and producing signals representative of the sensed near-infrared light;
   using the signals to determine an AC component of a first tissue oxygen parameter that is isolated from a DC component of the first tissue oxygen parameter;
   using the signals to determine an AC component of a second tissue oxygen parameter that is isolated from a DC component of the second tissue oxygen parameter; and
   determining a tissue arterial oxygen saturation value of a tissue body using the determined AC component of the first tissue oxygen parameter and the determined AC component of the second tissue oxygen parameter.

2. The method of claim 1, wherein the step of determining the tissue arterial oxygen saturation value includes determining a peak-to peak value of the AC component of the first tissue oxygen parameter and a peak-to-peak value of the AC component of the second tissue oxygen parameter.

3. The method of claim 1 or claim 2, wherein the first tissue oxygen parameter is oxyhemoglobin ($HbO_2$), and the second tissue oxygen parameter is deoxyhemoglobin (Hb).

4. The method of any preceding claim, wherein the step of determining the tissue arterial oxygen saturation value uses a ratio of the first tissue oxygen parameter and a sum of the first tissue oxygen parameter and the second tissue oxygen parameter.

5. The method of any preceding claim, wherein the step of using the signals to determine the AC component of the first tissue oxygen parameter includes using the signals to determine the first tissue oxygen parameter and subsequently identifying the AC component of the first tissue oxygen parameter isolated from the DC component of the first tissue oxygen parameter; and
   the step of using the signals to determine the AC component of the second tissue oxygen parameter includes using the signals to determine the second tissue oxygen parameter and subsequently identifying the AC component of the second tissue oxygen parameter isolated from the DC component of the first tissue oxygen parameter.

6. A method of non-invasively determining a tissue arterial oxygen saturation value of a tissue body, comprising:

   transmitting at least a first wavelength and a second wavelength of near-infrared light into a tissue body, the first wavelength different from the second wavelength;
   sensing the tissue body for the near-infrared light, and producing signals representative of the sensed near-infrared light;
   using the signals to determine an AC component of the signals that is isolated from a DC component of the signals;
   using the determined AC component of the signals to determine a first tissue oxygen parameter value and to determine a second tissue oxygen parameter value; and
   determining a tissue arterial oxygen saturation value of a tissue body using the determined first tissue oxygen parameter value and the determined second tissue oxygen parameter value.

7. The method of claim 6, wherein the step of using the determined AC component of the signals to determine the first tissue oxygen parameter value and to determine the second tissue oxygen parameter value includes determining a peak-to peak value of the AC component of the signals.

8. The method of claim 6 or claim 7, wherein the first tissue oxygen parameter is oxyhemoglobin ($HbO_2$), and the second tissue oxygen parameter is deoxyhemoglobin (Hb).

9. The method of any of claims 6 to 8, wherein the step of determining the tissue arterial oxygen saturation value uses a ratio of the first tissue oxygen parameter and a sum of the first tissue oxygen parameter and the second tissue oxygen parameter.

10. The method of any of claims 6 to 9, wherein the step of using the signals to determine the AC component of the signals that is isolated from the DC component of the signals includes filtering the signals to remove the DC component of the signals.

11. The method of any of claims 6 to 10, wherein the step of determining the tissue arterial oxygen saturation value of a tissue body includes using a peak-to-peak amplitude value of the determined first tissue oxygen parameter value.

12. The method of claim 11, wherein the step of determining the tissue arterial oxygen saturation value of a tissue body includes using a peak-to-peak amplitude value of a sum of the determined first tissue oxygen parameter value and the determined first second tissue oxygen parameter value.

13. The method of claim 12, wherein the first tissue oxygen parameter is oxyhemoglobin ($HbO_2$), and the second tissue oxygen parameter is deoxyhemoglobin (Hb).

*FIG. 1*

*FIG. 2*

Light source,
20

Cable,
24

Near detector,
22,22A

Far detector,
22,22B

**FIG. 3A**

Attachment
skin side

20

22,22A

22,22B

24

18

Sensor side view

14

**FIG. 3B**

120

114

122

**FIG. 4**

*FIG. 5*

```
┌─────────────────────────────────┐
│     NON-INVASIVELY SENSE SUBJECT │
│      TISSUE USING NEAR-INFRARED  │
│        SPECTROSCOPY (NIRS) AT    │
│   WAVELENGTHS CORRESPONDING TO   │
│     PARAMETERS (e.g., Hb, HbO2)  │
└─────────────────────────────────┘
                 │
                 ▼
┌─────────────────────────────────┐
│     PROCESS SIGNALS FROM NIRS    │
│        SENSING TO ISOLATE        │
│     PULSATILE (AC) COMPONENT     │
│           OF SIGNALS             │
└─────────────────────────────────┘
                 │
                 ▼
┌─────────────────────────────────┐
│     DETERMINE TISSUE OXYGEN      │
│        PARAMETER VALUES          │
│       (Hb, HbO2, THb, etc.)      │
│         USING ISOLATED           │
│           AC SIGNALS             │
└─────────────────────────────────┘
                 │
                 ▼
┌─────────────────────────────────┐
│       DETERMINE PEAK-TO-PEAK     │
│   AMPLITUDE OF PARAMETER VALUES  │
└─────────────────────────────────┘
                 │
                 ▼
┌─────────────────────────────────┐
│     DETERMINE ARTERIAL TISSUE    │
│   OXYGEN PARAMETER (e.g., SaO2)  │
│   USING PEAK-TO-PEAK AMPLITUDES  │
└─────────────────────────────────┘
```

*FIG. 6*

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63178120
- US 20140171761 A
- US 8428674 B
- US 7072701 B
- US 8396526 B
- US 8788004 B
- US 10918321 B

**Non-patent literature cited in the description**

- **ITO et al.** Arterial fraction of cerebral blood volume in humans measured by positron emission tomography. *Ann Nucl Med*, April 2001, vol. 15 (2), 111-6
- **PANG CC**. Measurement of body venous tone. *J Pharmacol Toxicol Methods*, 2000, vol. 44 (2), 341-60